# EUROPÄISCHE PATENTANMELDUNG

(11) **EP 2 716 680 A1**
(43) Veröffentlichungstag der Anmeldung: **09.04.2014**
(21) Anmeldenummer: 12187200.6
(22) Anmeldetag: 04.10.2012
(51) Int. Cl.: C08G 65/00

(54) **Fluorierte polymerisierbare Verbindung**

(71) Anmelder: BASF SE, 67056 Ludwigshafen (DE)
(72) Erfinder: Niedermair, Fabian, 83308 Trostberg (DE); Wolfertstetter, Franz, 83349 Palling (DE); Steidl, Norbert, Dr., 83361 Kienberg (DE); Burkhardt, Walther, Dr., 84518 Garching an der Alz (DE)
(74) Vertreter: Witz, Michael

(57) **Zusammenfassung**

Die Erfindung betrifft eine polymerisierbare Verbindung der Formel (I), wobei R_{f} für ein monovalentes fluoriertes organisches Radikal mit 1 bis 18 C-Atomen und 35 bis 85 Gew.-% Fluor, R¹ für einen Rest aus der von acyclischem oder cyclischem Alkylen mit 5 bis 14 Kohlenstoffatomen, Arylen, Diarylenalkan und dialkylensubstituiertem Aryl gebildeten Gruppe, B für -O- oder -N(R²)-, R² für Wasserstoff, einen aliphatischen Kohlenwasserstoffrest mit 1 bis 12 C-Atomen oder ein monovalentes fluoriertes organisches Radikal mit 1 bis 18 C-Atomen und 35 bis 85 Gew.-% Fluor, X für ein polymeres Polyol mit mindestens zwei Wiederholungseinheiten und nach Entfernen von zwei OH-Gruppen, E für -O- oder -N(R³)-, R³ für einen Kohlenwasserstoffrest mit 1 bis 12 C-Atomen oder für ein Wasserstoffatom, Y für eine chemische Bindung oder einen divalenten Rest mit 2 bis 20 C-Atomen und Z für einen organischen Rest mit einer polymerisierbaren Baugruppe steht. Weiterhin wird ein Verfahren zur Herstellung dieser Verbindung, Polymere umfassend die erfindungsgemäße polymerisierbare Verbindung sowie die Verwendung der Polymere zur Beschichtung einer Oberfläche offenbart.

## Beschreibung

Die vorliegende Erfindung betrifft fluorierte polymerisierbare Verbindungen. Weiterhin wird ein Verfahren zur Herstellung dieser Verbindung, Polymere umfassend die erfindungsgemäße polymerisierbare Verbindung sowie deren Verwendung zur Beschichtung von Oberflächen offenbart.

Beschichtungsmittel auf der Basis fluorhaltiger Polymere sind für viele sehr unterschiedliche Anwendungszwecke seit langem bekannt.

So sind beispielsweise in der EP-B-856 020 nichtwässrige Klarlacke auf der Basis fluorhaltiger Polyacrylat-Bindemittel und Polyisocyanatgruppenhaltiger Vernetzungsmittel beschrieben, die zu öl-, schmutz- und wasser-abweisenden Beschichtungen gehärtet werden können und die für die Beschichtung von Fahrzeugen, wie U-Bahnen, Zügen, Bussen u.Ä., industriellen Anlagen, wie z.B. Tanks, Gebäuden und anderen Bauwerken eingesetzt werden können. Insbesondere dienen die Klarlacke zur Beschichtung von Gebäuden, Bussen, Zügen und anderen Objekten, bei denen Graffiti ein Problem darstellt, da die resultierenden Beschichtungen leicht zu reinigen sind und außerdem eine gute Beständigkeit gegen Schmutz aufweisen. Zur Herstellung der fluorhaltigen Polyacrylat-Bindemittel werden ethylenisch ungesättigte Verbindungen mit Fluoralkylgruppen, wie beispielsweise Fluoroalkylethyl(meth)acrylate oder 2-(N-Ethylperfluoro-octan-sulfoamido)ethylmethacrylat, eingesetzt. Diese fluorhaltigen Monomere müssen allerdings separat und getrennt von den übrigen Monomeren zudosiert werden, so dass die Herstellung der Bindemittel recht aufwändig ist.

Ferner sind aus der EP-B-856 022 ähnliche Beschichtungsmittel bekannt, die zusätzlich zu fluorhaltigen Polyacrylat-Bindemitteln als Vernetzer Polyisocyanate enthalten, bei denen 0,1 bis 33 Mol-% der Isocyanatgruppen mit Perfluoralkoholen umgesetzt worden sind. Bei der dort beschriebenen Herstellung der fluorierten Polyisocyanate sind neben monofluorierten Polyisocyanaten auch hohe Anteile an di- und tri-fluorierten Polyisocyanaten zu erwarten, die an die Lackoberfläche migrieren und so den optischen Gesamteindruck (das sogenannte "Appearance"), die Härte, die Chemikalienbeständigkeit und andere mechanisch-technologische Eigenschaften der resultierenden Beschichtung negativ beeinflussen können.

Fluorhaltige Klarlacke sind auch in der WO05/030892 beschrieben, wobei diese Klarlacke als Bindemittel fluorhaltige Polyacrylate enthalten, die zusätzlich noch Organosilanmonomere einpolymerisiert enthalten. Die fluorhaltigen Polyacrylate werden wiederum erhalten durch Einpolymerisieren von ethylenisch ungesättigten Verbindungen mit Fluoralkylgruppen, wie beispielsweise Fluoroalkylethyl(meth)acrylate oder 2-(N-Ethylperfluorooctan-sulfoamido)alkyl(meth)acrylat.

Aus der WO05/030891 sind fluorierte Klarlacke mit verbesserter Klarlack/Klarlack-Haftung bekannt, die neben einem fluorierten Silanpolymer auf Basis von einpolymerisierten ethylenisch ungesättigten Verbindungen mit Fluoralkylgruppen, wie beispielsweise Fluoroalkylethyl(meth)acrylaten oder 2-(N-Ethylperfluorooctan-sulfoamido)alkyl(meth)acrylaten, zur Haftungsverbesserung ein fluoriertes Polyurethanharz enthalten. Dieses ist erhältlich durch Umsetzung eines Polyisocyanates mit einem perfluorierten Monoalkohol und einem oligomeren und/oder polymeren Polyetherpolyol, wie ethoxyliertes/propoxyliertes Glykol, und weist keine freien Isocyanatgruppen mehr auf.

Die WO05/080465 beschreibt leicht zu reinigende, abriebfeste und alkalibeständige Beschichtungen, die durch Verwendung von Beschichtungsmitteln erhalten werden, welche neben einem härtbaren Bindemittelsystem und anorganischen Teilchen mindestens ein fluorhaltiges Polymer oder Oligomer mit mindestens einer funktionellen Gruppe enthalten, die mit einer funktionellen Gruppe des Bindemittelsystems reaktionsfähig ist. Als fluorhaltiges Polymer oder Oligomer werden beispielsweise fluorierte Polyether, fluorierte Epoxide, fluorierte Polyurethane und fluorhaltige Polymerisate eingesetzt, wobei die fluorhaltigen Polymerisate unter Verwendung von handelsüblichen Fluormonomeren, wie Tetrafluorethylen, Vinylidenflourid u.Ä., hergestellt werden.

Die WO07/115752 beschreibt zweikomponentige wässrige Hybridreaktivharzsysteme auf Polyurethanbasis mit einem Epoxid-/Amin-Härtungsmechanismus, die im Bau- und Industriebereich zur Herstellung von mechanisch hoch belastbaren und einfach zu reinigenden Beschichtungen eingesetzt werden. Ggf. können diese Beschichtungsmittel amino- und/oder hydroxy- und/oder mercapto-funktionelle fluormodifizierte Makromonomere oder Telechele enthalten.

Ferner beschreibt die WO05/007762 wässrige ggf. fluorierte Polyurethanhybrid-Dispersionen mit kovalent gebundenen fluorierten Seitenketten, die über die Polyurethanbasis und/oder über radikalisch polymerisierbare fluorhaltige Monomere eingeführt werden können. Unter Verwendung dieser Polyurethanhybrid-Dispersionen lassen sich, bedingt durch die hohe Vernetzungsdichte in Verbindung mit einer hohen Härte, schmutzabweisende Beschichtungen mit guten mechanischen Eigenschaften und einer guten Lösemittel- und Chemikalienbeständigkeit herstellen, die für sehr viele verschiedene Einsatzzwecke geeignet sind.

Auch aus der WO08/040428 sind fluormodifizierte Polyurethanbeschichtungen insbesondere im Bau- und Industriebereich zur permanenten öl-, wasser- und schmutzabweisenden Beschichtung von mineralischen und nichtmineralischen Untergründen bekannt.

Weiterhin sind aus der EP-A-2 085 442 wässrige Beschichtungsmittel auf der Basis von Fluorsilan-Komponenten bekannt, die zur permanenten öl- und wasserabweisenden Oberflächenbehandlung von mineralischen und nichtmineralischen Untergründen für verschiedene Anwendungszwecke eingesetzt werden.

Aus der EP-B-587 667 sind Beschichtungsmittelzusammensetzungen auf der Basis von fluorhaltigen anorganischen Polykondensaten bekannt, die zur Beschichtung von Glas, Keramik, Metall, Kunststoffen und Papier, insbesondere zur Beschichtung von Außen- und Innenspiegeln sowie Windschutzscheiben von Kraftfahrzeugen, eingesetzt werden.

Die EP-A-1 844 863 beschreibt ebenfalls Beschichtungsmittel zur Herstellung stark flüssigkeitsabweisender Beschichtungen, also Beschichtungen, die mit einer Referenzflüssigkeit, wie insbesondere Wasser, einen möglichst großen Kontaktwinkel von 120° bis 180° ausbilden. Hierzu werden Beschichtungsmittel eingesetzt, die ein Polymer und ein keramisches Material bzw. Nanopartikel enthalten und die so zu Beschichtungen mit einer strukturierten Oberfläche führen. Angaben zur genaueren Zusammensetzung der Polymere sind jedoch nicht enthalten.

Die WO96/04123 beschreibt hydrophobe Beschichtungsmittel zur Herstellung selbstreinigender Oberflächen, bei denen eine künstliche Oberflächenstruktur aus Erhebungen und Vertiefungen erzeugt wird und die Erhebungen aus hydrophobierten Materialien bestehen. Hierzu wird beispielsweise Teflon verwendet.

Für Oberflächenbeschichtungen wurden bereits Versuche mit kammförmigen Polymeren mit fluorierten Seitenketten durchgeführt. Diesbezüglich ist bekannt, dass zur Erzielung einer niedrigen Oberflächenenergie und somit guten schmutz- und wasserabweisenden Eigenschaften möglichst lange fluorierte Ketten und möglichst kurze Abstandshalter ("Spacer") zur Hauptkette, in Form von Kohlenwasserstoffgruppen, entscheidend sind. Längere Spacer führen hingegen zu deutlich schlechteren Ergebnissen ("Fluorinated comblike homopolymers: The effect of spacer lengths on surface properties", Saïdi, Salima; Guittard, Frédéric; Guimon, Claude; Géribaldi, Serge; Journal of Polymer Science Part A: Polymer Chemistry, vol. 43, issue 17, pp. 3737-3747).

Aufgabe der vorliegenden Erfindung war es daher, die bekannten Beschichtungsmittel auf Basis fluorhaltiger Polymere weiter zu verbessern. Insbesondere sollten fluorierte Monomere zur Verfügung gestellt werden, welche auch in geringen Einsatzmengen und bei einem geringeren Fluor-Gehalt, in den daraus hergestellten Beschichtungen zu hervorragenden wasser- und schmutzabweisenden Eigenschaften führen.

Ferner sollten fluorierte Monomere zur Verfügung gestellt werden, die in den daraus hergestellten Produkten eine sehr gute mechanische und photochemische Beständigkeit sowie einen guten optischen Gesamteindruck (sogenanntes "Appearance"), eine möglichst hohe Härte und eine gute Chemikalienbeständigkeit gewährleisten. Außerdem sollten die fluorierten Monomere möglichst einfach und kostengünstig herstellbar sein.

Gelöst wurde diese Aufgabe durch eine polymerisierbare Verbindung der Formel (I), 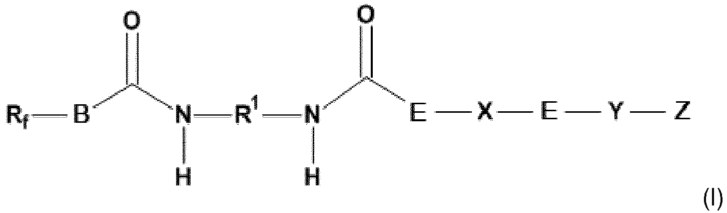 wobei
- R_{f}: für ein monovalentes fluoriertes organisches Radikal mit 1 bis 18 C-Atomen und 35 bis 85 Gew.-% Fluor,
- R¹: für einen Rest aus der von acyclischem oder cyclischem Alkylen mit 5 bis 14 Kohlenstoffatomen, Arylen, Diarylenalkan und dialkylensubstituiertem Aryl gebildeten Gruppe
- B: für -O- oder -N(R²)-
- R²: für Wasserstoff, einen aliphatischen Kohlenwasserstoffrest mit 1 bis 12 C-Atomen oder ein monovalentes fluoriertes organisches Radikal mit 1 bis 18 C- Atomen und 35 bis 85 Gew.-% Fluor
- X: für ein polymeres Polyol mit mindestens zwei Wiederholungseinheiten und nach Entfernen von zwei OH-Gruppen
- E: für -O- oder -N(R³)-
- R³: für einen Kohlenwasserstoffrest mit 1 bis 12 C-Atomen oder für ein Wasserstoffatom,
- Y: für eine chemische Bindung oder einen divalenten Rest mit 2 bis 20 C-Atomen und
- Z: für einen organischen Rest mit einer polymerisierbaren Baugruppe steht.

Überraschend hat sich hierbei ergeben, dass nicht nur die gestellte Aufgabe in vollem Umfang gelöst werden konnte, sondern dass die Monomere auch in einfacher Weise zu Polymeren umgesetzt werden können. Weiterhin kann bei der Herstellung der erfindungsgemäßen Monomere auf die Verwendung von Lösungsmittel vollständig verzichtet werden.

Insbesondere war im Hinblick auf den Stand der Technik überraschend, dass trotz des vorhandenen Spacers zwischen dem fluorierten organischen Rest R_{f} und der polymerisierbaren Baugruppe Z sehr gute schmutz- und wasserabweisende Eigenschaften der hieraus hergestellten Polymeren erhalten werden.

Besonders bevorzugt steht die Gruppe X für ein polymeres Polyol mit mindestens zwei Wiederholungseinheiten und nach Entfernen von zwei OH-Gruppen, ausgewählt aus der Gruppe Polyester, Polyalkylenether, Polycarbonate, Polyestercarbonate, Polyacetale, Polyurethanpolyole oder Polysiloxanpolyol. Bevorzugte polymere Polyole sind Polyester, Polyalkylenether, Polyestercarbonate und Polycarbonate.

Geeignete polymere Polyole nach Entfernung von zwei OH-Gruppen liegen bevorzugt im Molgewichtsbereich von 72 bis 3000, bevorzugt von 100 bis 2000 und besonders bevorzugt von 150 bis 1000.

Die erfindungsgemäßen Polyalkylenether können durch die allg. Strukturformel (II) wiedergegeben werden, wobei R⁴ gleich oder verschieden sein kann und n eine ganze Zahl von 1 bis 70, bevorzugt von 2 bis 40 und insbesondere von 2 bis 20 repräsentiert.

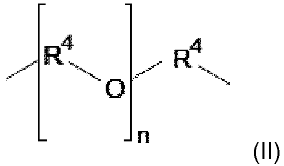

Erfindungsgemäß kann es sich beispielsweise um Polyalkylenglykole wie Polyethylenglykole, Polypropylenglykole und Polyepichlorhydrine sowie Epoxidharze, Polytetrahydrofurane, Polyoxetane, Polyphenylenether oder Polyetherketone handeln. Unter den Begriffen Polyalyklenglykole versteht man überwiegend lineare Polyether, d. h. Polymere mit endständigen Hydroxy-Gruppen. Die erfindungsgemäß bevorzugten Vertreter dieser Polyether-Polyole sind die Polyethylenglykole, Polypropylenglykole bzw. Polytetramethylenglykole, die durch Polyaddition, insbesondere mittels KOH- oder DMC-Katalyse, von Ethylenoxid, Propylenoxid bzw. Tetrahydrofuran an Wasser hergestellt werden. Weiterhin bevorzugt sind Blockcopolymere aus Ethylen- und Propylenoxid.

Insbesondere kann es sich bei dem Polyalkylenether um eine Baugruppe mit der Formel (III)

-(CₘH₂ₘO)ₐ-(CₘH₂ₘ)- (III)

handeln, wobei m unabhängig voneinander für jede (CₘH₂ₘO)-Einheit gleich oder verschieden für eine ganze Zahl zwischen 2 und 18 und a für eine ganze Zahl zwischen 1 und 30 steht.

Die erfindungsgemäß einsetzbaren Polyalkylenether mit C₅-C₁₀₀-Alkylengruppen können mittels Polykondensationsreaktionen aus Diolen in Gegenwart von Sulfobernsteinsäure als Katalysator hergestellt werden.

Als Diol-Komponente geeignet sind insbesondere gesättigte oder ungesättigte, verzweigte oder unverzweigte aliphatische Dihydroxyverbindungen mit 5 bis 36 Kohlenstoffatomen oder aromatische Dihydroxy-Verbindungen, wie beispielsweise 1,5-Pentandiol, 1,6-Hexandiol, Neopentylglykol, Bis-(hydroxymethyl)cyclohexane, Bisphenol A, Dimerdiole, hydrierte Dimerdiole oder auch Mischungen der genannten Diole.

Bei dem erfindungsgemäßen polymeren Polyol kann es sich um Polyalkylenether, die durch Polykondensation eines Dimerdiols oder α,ω-C₅-C₃₆-Alkandiols gewonnen werden, handeln. Die Polykondensation wird vorzugsweise in Gegenwart von Sulfobernsteinsäure durchgeführt, kann aber ebenso mit anderen gleichwirkenden Katalysatoren vorgenommen werden. Dimerdiole sind herstellungsbedingt Gemische; ihre Herstellung ist hinreichend aus dem Stand der Technik bekannt, beispielsweise gemäß DE 1 768 313 und US 2,347,562. Als Dimerdiol-Komponenten für die Umsetzung zu den erfindungsgemäß einsetzbaren Polyalkylenethern eignen sich vorzugsweise Dimerdiole mit einer Gesamtkohlenstoffzahl von C₁₂-C₁₀₀. Besonders gut geeignet sind C₁₂-C₄₀-Dimerdiole, vorzugsweise C₁₂-C₂₀-und besonders bevorzugt C₁₂-C₁₆-Dimerdiole, wobei sich die Angabe der C-Kettenlänge hier auf eine Kette bezieht.

Besonders bevorzugt steht die Gruppe X für ein polymeres Polyol mit mindestens zwei Wiederholungseinheiten und nach Entfernen von zwei OH-Gruppen auf Lacton-Basis. Hierbei kann es sich um Homo- oder Mischpolymerisate von Lactonen, bevorzugt um endständige Hydroxylgruppen aufweisende Anlagerungsprodukte von Lactonen an geeignete difunktionelle Startermoleküle handeln. Beispiele für geeignete Lactone sind [ε]-Caprolacton, [β]-Propiolacton, [γ]-Butyrolacton und/oder Methyl-[ε]-caprolacton sowie deren Gemische. Anstelle der Polymerisate von Lactonen können auch insbesondere bevorzugt die entsprechenden chemisch äquivalenten Polykondensate der den Lactonen entsprechenden Hydroxycarbonsäuren eingesetzt werden.

In einer bevorzugten Ausführungsform kann es sich somit bei der Gruppe X um einen Polyester handeln. Insbesondere kann es sich bei dem Polyester um eine Baugruppe mit der Formel (IV) 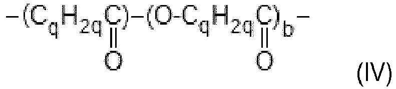 handeln, wobei q unabhängig voneinander für jede (C_{q}H_{2q}(C=O))-Einheit gleich oder verschieden für eine ganze Zahl zwischen 2 und 18 und b für eine ganze Zahl zwischen 1 und 30 steht.

Weiterhin kann die Gruppe X für ein polymeres Polyol, nach Entfernung von zwei OH-Gruppen mit mindestens zwei Wiederholungseinheiten, stehen, welches aus der Gruppe der Hydroxylgruppen aufweisenden Polycarbonate ausgewählt ist. In Frage kommen hierbei Verbindungen, welche durch Reaktion von Kohlensäurederivaten, z. B. Diphenylcarbonat, Dimethylcarbonat oder Phosgen, mit Diolen erhältlich sind. Besonders eignen sich als Diole z.B. Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol, 1,3-Dipropylenglykol, Polypropylenglykole, Dibutylenglykol, Polybutylenglykole, Bisphenol A und Tetrabrombisphenol A.

Es ist erfindungswesentlich dass R¹ in Formel (I) für einen Rest aus der von acyclischem oder cyclischem Alkylen mit 5 bis 14 Kohlenstoffatomen, Arylen, Diarylenalkan und dialkylensubstituiertem Aryl gebildeten Gruppe steht. In einer bevorzugten Ausführungsform ist R¹ ausgewählt aus der Gruppe Methylen-, Ethylen-, Propylen-, Butylen-, Pentamethylen-, Hexamethylen-, Octamethylen-, 2-Ethylhexamethylen-, Phenylen-, Toluylen-, Methylenbisphenyl-, Propylenbisphenyl-, Cyclohexylen-, Methylen- oder Propylenbiscyclohexyl-Reste und einer Gruppe die sich durch Entfernen der Isocyanatgruppen aus 1-Isocyanato-3,3,5-trimethyl-5-isocyanatomethyl-cyclohexan ergibt. Insbesondere bevorzugt handelt es sich bei R¹ um und eine Gruppe die sich durch Entfernen der Isocyanatgruppen aus 1-Isocyanato-3,3,5-trimethyl-5-isocyanatomethylcyclohexan ergibt.

Die Gruppe Y steht für eine chemische Bindung oder einen divalenten Rest mit 2 bis 20 C-Atomen. Wenn es sich bei Y nicht um eine chemische Bindung handelt, wird Y bevorzugt ausgewählt aus der Gruppe Methylen-, Ethylen-, Propylen-, Butylen-, Pentamethylen-, Hexamethylen-, Octamethylen-, 2-Ethylhexamethylen-, Phenylen-, Toluylen-, Methylenbisphenyl-, Propylenbisphenyl-, Cyclohexylen-, Methylen- oder Propylenbiscyclohexyl-Reste und einer Gruppe die sich durch Entfernen der Isocyanatgruppen aus 1-Isocyanato-3,3,5-trimethyl-5-isocyanatomethyl-cyclohexan ergibt. Insbesondere bevorzugt handelt es sich bei Y um eine Gruppe, die sich durch Entfernen der Isocyanatgruppen aus 1-Isocyanato-3,3,5-trimethyl-5-isocyanatomethyl-cyclohexan ergibt.

Bevorzugt handelt es sich bei R_{f} um eine Gruppe die sich durch Entfernen der Hydroxygruppe aus mindestens einer der folgenden Verbindungen ergibt: 3,3,4,4,5,5,6,6,7,7,8,8,8-Tridecafluoroctan-1-ol, 3,3,4,4,5,5,6,6,7,7,8,8,9,9,10,10,10-heptadecafluordecan-1-ol, 3,3,4,4,5,5,6,6,7,7,8,8,9,9,10,10,11,11,12,12,12-heneicosafluordodecan-1-ol, 3,3,4,4,5,5,6,6,7,7,8,8,9,9,10,10,11,11,12,12,13,13,14,14,14-pentacosafluortetradecan-1-ol, 3,3,4,4,5,5,6,6,7,7,8,8,9,9,10,10,11,11,12,12,13,13,14,14,15,15,16,16,16-nonacosafluorhexadecan-1-ol, 3,3,4,4,5,5,6,6,7,7,8,8-Dodecafluorheptan-1-ol, 3,3,4,4,5,5,6,6,7,7,8,8,9,9,10,10-Hexadecafluornonan-1-ol, 3,3,4,4,5,5,6,6,7,7,8,8,9,9,10,10,11,11,12,12-eicosafluorundecan-1-ol, 3,3,4,4,5,5,6,6,7,7,8,8,9,9,10,10,11,11,12,12,13,13,14,14-tetracosafluortridecan-1-ol, 3,3,4,4,5,5,6,6,7,7,8,8,9,9,10,10,11,11,12,12,13,13,14,14,15,15,16,16-octacosafluorpentadecan-1-ol, 4-(3,3,4,4,5,5,6,6,7,7,8,8,8-Tridecafluoroctyl)-benzylalkohol, 4-(3,3,4,4,5,5,6,6,7,7,8,8,9,9,10,10,10-Heptadecafluordecyl)-benzylalkohol, 4-(4,4,5,5,6,6,7,7,8,8,9,9,9-Tridecafluornonyloxy)-benzylalkohol, 4-(4,4,5,5,6,6,7,7,8,8,9,9,10,10,11,11,11-Heptadecafluorundecyloxy)-benzylalkohol sowie die Handelsprodukte Fluowet® EA 600, Fluowet® EA 800, Fluowet® EA 093, Fluowet® EA 612, Fluowet® EA 612 N, Fluowet® EA 812 AC, Fluowet® EA 812 IW, Fluowet® EA 812 EP, Fluowet® EA 6/1020, Fluowet® PA, bestehend aus Perfluoralkylethanol-Gemischen, Fluowet® OTL, Fluowet® OTN, bestehend aus ethoxylierten Perfluoralkylethanol-Gemischen, der Fa. Clariant GmbH, die Handelsprodukte A-1620, A-1630, A-1660, A-1820, A-1830, A-1860, A-2020, A-3620, A-3820, A-5610, A-581 0 der Fa. Daikin Industries, Ltd., die Handelsprodukte Zonyl® BA, Zonyl® BA L, Zonyl® BA LD, Foralkyl® EOH-6N LW, bestehend aus Perfluoralkylethanol-Gemischen, Zonyl® OTL, Zonyl® OTN, bestehend aus ethoxylierten Perfluoralkylethanol-Gemischen, Zonyl® FSH, Zonyl® FSO, Zonyl® FSN, Zonyl® FS-300, Zonyl® FSN-100, Zonyl® FSO-1 00 der Fa. Du Pont de Nemours, die Handelsprodukte Krytox® der Fa. Du Pont de Nemours, bestehend aus Hexafluorpropenoxid (HFPO)-Oligomer-Alkohol-Gemischen, oder geeignete Kombinationen daraus. Vorzugsweise Perfluoralkylethanol-Gemische mit 30 - 49,9 Gew.-% an 3,3,4,4,5,5,6,6,7,7,8,8,8-Tridecafluoroctan-1-ol und 30 - 49,9 Gew.-% an 3,3,4,4,5,5,6,6,7,7,8,8,9,9,10,10,10-heptadecafluordecan-1-ol wie die Handelsprodukte Fluowet® EA 612 und Fluowet® EA 812. Außerdem kommen die Handelsprodukte A-1620, A-1820 der Fa. Daikin Industries, Ltd. in Frage.

Weiterhin bevorzugt wird zur Einführung des Restes R_{f} ein Amin, insbesondere R_{f}NH₂ eingesetzt. Bevorzugt handelt es sich bei R_{f} somit um eine Gruppe, die sich durch Entfernen der Aminogruppe aus mindestens einer der folgenden Verbindungen ergibt: 3,3,4,4,5,5,6,6,7,7,8,8,8-Tridecafluoroctylamin, 3,3,4,4,5,5,6,6,7,7,8,8,9,9,10,10,10-heptadecafluordecylamin, 3,3,4,4,5,5,6,6,7,7,8,8,9,9,10,10,11,11,12,12,12-heneicosafluordodecylamin, 3,3,4,4,5,5,6,6,7,7,8,8,9,9,10,10,11,11,12,12,13,13,14,14,14-pentacosafluortetradecylamin, 3,3,4,4,5,5,6,6,7,7,8,8,9,9,10,10,11,11,12,12,13,13,14,14,15,15,16,16,16-nonacosafluorhexadecylamin, 4-(3,3,4,4,5,5,6,6,7,7,8,8,8-Tridecafluoroctyl)-benzylamin, 4-(3,3,4,4,5,5,6,6,7,7,8,8,9,9,10,10,10-Heptadecafluordecyl)-benzylamin, sowie 1,1,1,2,2,3,3,4,4,5,5,6,6-Tridecafluor-8-iodoctan, 1,1,1-2,2,3,3,4,4,5,5,6,6,7,7,8,8-Heptadecafluor-10-ioddecan, 1,1,1,2,2,3,3,4,4,5,5,6,6,7,7,8,8,9,9,10,10-heneicosafluor-12-ioddodecan, 1,1,1,2,2,3,3,4,4,5,5,6,6,7,7,8,8,9,9,10,10,11,11,12,12-pentacosafluor-14-iodtetradecan, 1,1,1,2,2,3,3,4,4,5,5,6,6,7,7,8,8,9,9,10,10,11,11,12,12,13,13,14,14-nonacosafluor-16-iodhexadecan, den Handelsprodukten Fluowet® I 1600, Fluowet® I 800, Fluowet® 1612, Fluowet® 1812, Fluowet® 16/1020, Fluowet® 11020, bestehend aus Perfluoralkyliodid-Gemischen, Fluowet® EI 600, Fluowet® EI 800, Fluowet® EI 812, Fluowet® EI 6/1020, bestehend aus Perfluoralkylethyliodid-Gemischen, der Fa. Clariant GmbH und geeigneten Aminierungsreagentien, die Handelsprodukte U-1610, U-1710, U-1810 der Fa. Daikin Industries, Ltd. oder geeignete Kombinationen daraus eingesetzt werden. Bevorzugt werden Perfluoralkylethanol-Gemische mit 30 - 49,9 Gew.-% an 3,3,4,4,5,5,6,6,7,7,8,8,8-Tridecafluoroctylamin und 30 - 49,9 Gew.-% an 3,3,4,4,5,5,6,6,7,7,8,8,9,9,10,10,10-heptadecafluordecylamin eingesetzt.

In einer besonders bevorzugten Ausführungsform handelt es sich bei R_{f} um mindestens einen Rest der allgemeinen Formel (V),

CF₃-(CF₂)_{z}-(CH₂)_{y}- (V)

wobei z gleich oder verschieden für eine ganze Zahl von 0 bis 16 und y gleich oder verschieden für eine ganze Zahl von 1 bis 6 steht.

Besonders bevorzugt handelt es sich bei R_{f} um einen Rest mit der Formel CF₃-(CF₂)₅-(CH₂)₂-.

Bei Z kann es sich es sich um eine Vielzahl verschiedener organischer Reste handeln, wobei diese lediglich die Voraussetzung erfüllen müssen, eine polymerisierbare Baugruppe zu umfassen.

Bevorzugt handelt es sich bei Z um mindestens einen Rest aus der Reihe Acrylat, Methacrylat, Oxyalkylacrylat, Oxyalkylmethacrylat, Dialkanolaminyl, Oxyalkylcaprolactonacrylat, Oxyalkylcaprolactonmethacrylat, Oxycyloalkylmethacrylat, Oxycyloalkylacrylat und Oxyalkylmaleinsäureimid, bevorzugt Oxyalkylmethacrylat, Dialkanolaminyl und Oxyalkylcaprolactonmethacrylat.

In einer besonders bevorzugten Ausführungsform kann es sich bei der polymerisierbaren Verbindung der Formel (I) um eine Verbindung aus der Reihe (VI), (VII), (VIII) oder (IX) handeln, welche durch folgende Formel repräsentiert werden: 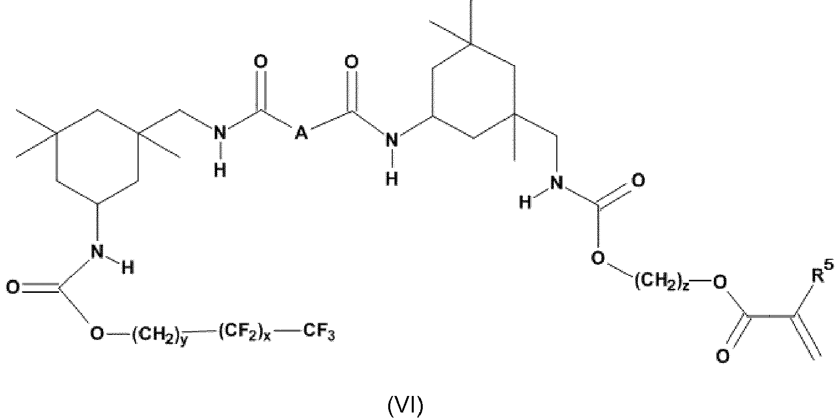 wobei
- A: für eine Baugruppe mit der Formel -O-(CₘH₂ₘO)ₐ-, mit m unabhängig voneinander für jede (CₘH₂ₘO)-Einheit gleich oder verschieden für eine ganze Zahl von 2 bis 4 und a für eine ganze Zahl von 2 bis 30
- R⁵: für Wasserstoff oder Methyl
- z: für eine ganze Zahl von 1 bis 5
- x: für eine ganze Zahl von 0 bis 9
- y: für eine ganze Zahl von 2 bis 12 steht, 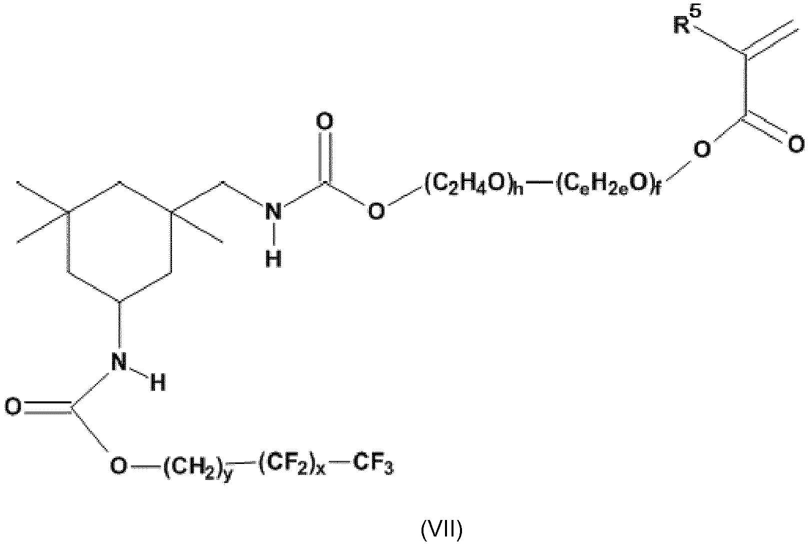
wobei
- R⁵: für Wasserstoff oder Methyl
- e: unabhängig voneinander für jede (CₑH₂ₑO)-Einheit gleich oder verschieden für 2 oder 3
- f: für eine ganze Zahl von 0 bis 150
- h: für eine ganze Zahl von 0 bis 50, wobei die Summe aus f und h mindestens 3 ist
- x: für eine ganze Zahl von 0 bis 9
- y: für eine ganze Zahl von 2 bis 12 steht, 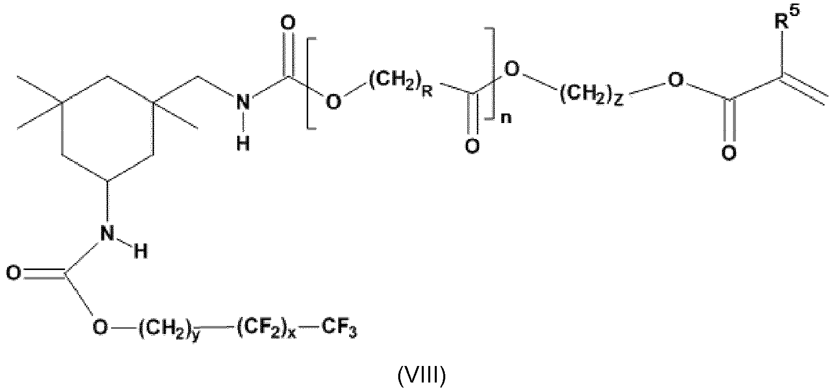
wobei
- Z: für eine ganze Zahl von 1 bis 50, insbesondere 1 bis 5,
- R: für eine ganze Zahl von 2 bis 18, insbesondere 2 bis 8,
- n: für eine Zahl von 1 bis 10
- x: für eine ganze Zahl von 0 bis 9
- y: für eine ganze Zahl von 2 bis 12
steht, 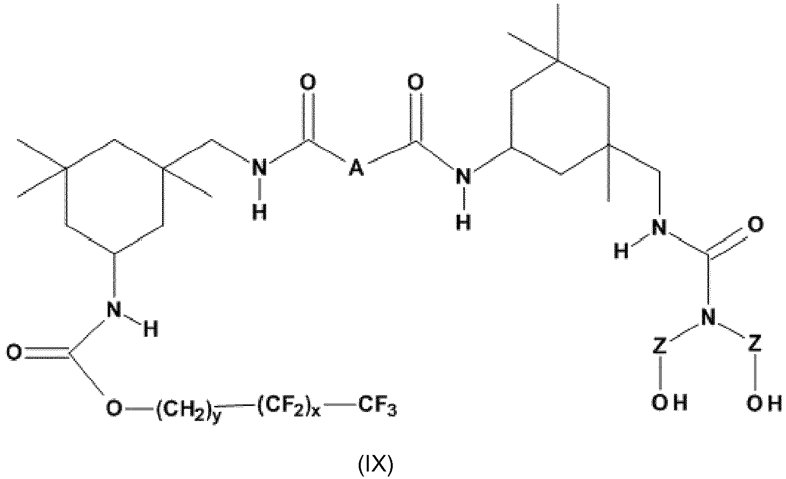 wobei
- A: für eine Baugruppe mit der Formel -O-(CₘH₂ₘO)ₐ-, mit m unabhängig voneinander für jede (CₘH₂ₘO)-Einheit gleich oder verschieden für eine ganze Zahl von 2 bis 4 und a für eine ganze Zahl von 2 bis 30
- Z: für eine (CₚH₂ₚ)-Einheit steht,
- p: für eine ganze Zahl von 2 bis 8
- x: für eine ganze Zahl von 0 bis 9
- y: für eine ganze Zahl von 2 bis 12
steht

Weiterhin wird durch die vorliegende Erfindung ein Verfahren zur Herstellung der erfindungsgemäßen polymerisierbaren Verbindung vorgesehen, wobei
(a) mindestens ein polymeres Polyol mit mindestens zwei Wiederholungseinheiten und mindestens zwei Hydroxygruppen mit mindestens einem Diisocyanat umgesetzt wird und das Produkt nachfolgend mit
(b) mindestens einem Alkohol oder Amin, mit einem fluorierten organischen Rest mit 1 bis 18 C-Atomen und 35 bis 85 Gew.-% Fluor, umgesetzt wird und nachfolgend das Produkt mit
(c) einer organischen Verbindung mit einer polymerisierbaren Baugruppe umgesetzt wird.

Besonders vorteilhaft kann die Reaktion in Abwesenheit von Lösungsmitteln durchgeführt werden. Dabei kann das eingesetzte polymere Polyol selbst als Lösungsmittel wirken, beispielsweise Polycarbonat-Diole mit einem Molekulargewicht von 1000 bis 2000 g/mol, PolyTHF mit einem Molekulargewicht von 1000 bis 2000 g/mol, insbesondere 1700 g/Mol und Polyethylenglycol mit einem Molekulargewicht von 500 bis 1500 g/mol, insbesondere 750 g/mol. Für niedermolekulare polymere Polyole, beispielsweise PolyTHF mit einem Molekulargewicht von 200 bis 700 g/mol, kann bevorzugt Proglyde DMM (Dipropylenglykol-Dimethylether) zusätzlich eingesetzt werden. Es ist hierbei jedoch besonders vorteilhaft, dass die Menge, falls überhaupt erforderlich, deutlich geringer ist als nach dem Stand der Technik.

In Schritt (a) wird als Diisocyanat bevorzugt eines aus der Reihe Hexamethylendiisocyanat-1,6 (HDI), 4,4'-, 2,4'- und/oder 2,2'-Diphenylmethandiisocyanat (MDI), 4,4'-Dicyclohexylmethandiisocyanat (H12MDI), Diphenylmethandiisocyanat (MDI), 2,4-und/oder 2,6-Toluylendiisocyanat (TDI) und 1-Isocyanato-3,3,5-trimethyl-5-isocyanatomethyl-cyclohexan (IPDI) eingesetzt. Weiterhin kann es sich um m-Xylendiisocyanat (MXDI), m- oder p-Tetramethylxylendiisocyanat (m-TMXDI, p-TMXDI), Naphthalin-1,5-Diisocyanat, Cyclohexan-1,4-diisocyanat, hydriertes Xylylen-diisocyanat (H6XDI), 1-Methyl-2,4-diisocyanato-cyclohexan, Tetramethoxybutan-1,4-diisocyanat, Butan-1,4-diisocyanat, 1,6-Diisocyanato-2,2,4-trimethylhexan, 1,6-Diisocyanato-2,4,4-trimethylhexan, 1-Isocyanato-1-methyl-4(3)-isocyanatomethylcyclohexan (IMCI) sowie 1,12-Dodecandiisocyanat (C12DI), 4-Dichlorophenyl diisocyanat, Dicyclohexylmethan-4,4'-diisocyanat, m-Phenylendiisocyanat, p-Phenylendiisocyanat, 4-Chloro-1,3-phenylendiisocyanat, 1,10-Decamethylenediisocyanat, Lysinalkylesterdiisocyanat, 3,3'-Dimethyl-4,4'-diphenylmethandiisocyanat, Xylylendiisocyanat, Tetramethylxylylendiisocyanat, 1,5-Tetrahydronaphthalendiisocyanat, Methylenbis(cyclohexyl)-2,4'-diisocyanat und 4-Methylcyclohexan-1,3-diisocyanat handeln.

Zur Herstellung der polymerisierbaren Verbindung der Formel (I) ist es besonders vorteilhaft Diisocyanate mit zwei unterschiedlich reaktiven Isocyanatgruppen, einzusetzen. Hierdurch ist gewährleistet, selektiv monofluorierte Umsetzungsprodukte zu erzeugen und entsprechend - falls überhaupt - nur einen äußerst geringen Anteil an bifluorierten und/oder nicht fluorierten Umsetzungsprodukten zu erhalten. Dies wiederum führt dazu, dass bei der folgenden Herstellung der polymerisierbaren Verbindung der Formel (I) weniger Nebenprodukte entstehen und die fluorierten Verbindungen nahezu vollständig in das Polymer eingebaut werden können und entsprechend auch in gehärteten Beschichtung in das Netzwerk eingebaut sind. Besonders bevorzugt ist deshalb der Einsatz von 1-Isocyanato-3,3,5-trimethyl-5-isocyanatomethyl-cyclohexan.

In Schritt (b) können insbesondere die hinsichtlich der Formel (I) beschriebenen fluorierten Alkohole und/oder fluorierten Amine eingesetzt werden.

In Schritt (c) handelt es sich bei dem polymeren Polyol wiederum bevorzugt um mindestens eines aus der Gruppe Gruppe Polyester, Polyalkylenether, Polycarbonate, Polyestercarbonate, Polyacetale, Polyurethanpolyole oder Polysiloxanpolyol. Bevorzugte polymere Polyole sind Polyester, Polyalkylenether, Polyestercarbonate und Polycarbonate. Geeignete polymere Polyole sind die hinsichtlich der Formel (I) beschriebenen polymeren Polyole.

Bei der organischen Verbindung mit einer polymerisierbaren Baugruppe handelt es sich bevorzugt um mindestens eine aus der Reihe Acrylsäure, Methacrylsäure, Hydroxyalkylacrylat, Hydroxyalkylmethacrylat, Dialkanolamin, Hydroxyalkylcaprolactonacrylat, Hydroxyalkylcaprolactonmethacrylat, Hydroxycyloalkylmethacrylat, Hydroxycyloalkylacrylat und Hydroxyalkylmaleinsäureimid, bevorzugt Hydroxyalkylmethacrylat, Dialkanolamin, Hydroxyalkylcaprolactonmethacrylat und Tert-Butylaminoethylmethacrylat.

Insbesondere bevorzugt sind Hydroxyethylmethacrylat und tert-Butylaminoethylmethacrylat.

Weiterhin sieht die vorliegende Erfindung ein alternatives Verfahren vor, wobei
(a) mindestens ein Alkohol oder Amin, mit einem fluorierten organischen Rest mit 1 bis 18 C-Atomen und 35 bis 85 Gew.-% Fluor, mit mindestens einem Diisocyanat umgesetzt wird und das Produkt nachfolgend mit
(b) mindestens ein polymeres Polyol, mit mindestens zwei Wiederholungseinheiten
sowie mit mindestens einer Hydroxygruppe und einem polymersierbaren Rest umgesetzt wird.

Bei dem polymeren Polyol, mit mindestens zwei Wiederholungseinheiten sowie mit mindestens einer Hydroxygruppe und einem polymersierbaren Rest kann es sich bevorzugt um Polyglykol-Methacrylate handeln.
Verfahren zur Herstellung von Polyglykol-Methacrylaten sind beispielsweise aus der DE19602035 bekannt. Besonders geeignete Polyglykol-Methacrylate sind wasserfreie Polyethylenglykol-Methacrylate mit Molekulargewichten zwischen 700 und 1000 g/mol.

Ein weiterer Gegenstand der vorliegenden Anmeldung ist ein Polymer, umfassend die erfindungsgemäßen polymerisierbaren Verbindungen. Insbesondere kann es sich hierbei um kammförmige Polymere handeln, wobei die polymerisierbare Gruppe in Formel (I) in die Hauptkette des Polymers eingebunden wird und der übrige Rest, umfassend die Gruppe R_{f}, als Seitenketten fungiert.

Bei dem Polymer, umfassend die erfindungsgemäßen polymerisierbaren Verbindungen, kann es sich insbesondere um Copolymere mit mindestens einem weiteren Monomer handeln. Als weitere Monomere kommen in Abhängigkeit von der polymerisierbaren Gruppe in Formel (I) verschiedene Verbindungen in Frage.

Handelt es sich bei der polymerisierbaren Gruppe um eine ungesättigte Doppelbindung, so kommen beispielsweise mindestens eine Verbindung aus der Reihe Ethyldiglycolacrylat, 4-tert. Butylcyclohexylacrylat, Dihydrocyclopentadienylacrylat, Lauryl(meth)acrylat, Phenoxyethyl(meth)acrylat, Isobornyl(meth)acrylat, Dimethylaminoethyl(meth)acrylat, Cyanoacrylate, Citraconat, Itaconat und deren Derivate, (Meth)acrylsäure, Methyl(meth)acrylat, Ethyl(meth)acrylat, n-Propyl(meth)acrylat, Isopropyl(meth)acrylat, n-Butyl(meth)acrylat, Isobutyl(meth)acrylat, tert-Butyl(meth)acrylat, n-Pentyl(meth)acrylat, n-Hexyl(meth)acrylat, Cyclohexyl(meth)acrylat, n-Heptyl(meth)acrylat, n-Octyl(meth)acrylat, 2-Propylheptyl(meth)acrylat, 2-Ethylhexyl(meth)acrylat, Nonyl(meth)acrylat, Decyl(meth)acrylat, iso Decyl(meth)acrylat, Dodecyl(meth)acrylat, Phenyl(meth)acrylat, Toluyl(meth)acrylat, Benzyl(meth)acrylat, 2-Methoxyethyl(meth)acrylat, 3-Methoxybutyl(meth)acrylat, 2-Hydroxyethyl(meth)acrylat, 2-Hydroxypropyl(meth)acrylat, Stearyl(meth)acrylat, Glycidyl(meth)acrylat, 2-Aminoethyl(meth)acrylate, γ-(Methacryloyloxypropyl)trimethoxysilan, Ethyleneoxid Addukte von (Meth)acrylsäure, Trifluoromethylmethyl(meth)acrylat, 2-Trifluoromethylethyl(meth)acrylat, 2-Perfluoroethylethyl(meth)acrylat, 2-Perfluoroethyl-2-perfluorobutylethyl(meth)acrylat, 2-Perfluoroethyl(meth)acrylat, Perfluoromethyl(meth)acrylat, Diperfluoromethylmethyl(meth)acrylat, 2-Perfluoromethyl-2-perfluoroethylmethyl(meth)acrylat, 2-Perfluorohexylethyl(meth)acrylat, 2-Perfluorodecylethyl(meth)acrylat und 2-Perfluorohexadecylethyl(meth)acrylat in Frage. Insbesondere bevorzugt sind Poly(ethylenglykol)methylether(meth)acrylat, (Meth)acrylsäure und Methylmethacrylat.

Handelt es sich bei der polymerisierbaren Gruppe um eine ungesättigte Doppelbindung, so kommen je nach Einsatzzweck weiterhin, z.B. Isoprenol oder Hydroxybutylvinylether in Frage. Weiterhin seien hier mono- und polyungesättigte Kohlenwasserstoffmonomere, Vinylester (bspw. Vinylester von C₁- bis C₆-gesättigten Monocarbonsäuren), Vinylether, monoethylenisch ungesättigte Mono- und Polycarbonsäuren und Alkylester dieser Mono- und Polycarbonsäuren (bspw. Acrylsäureester und Methacrylsäureester wie etwa C₁- bis C₁₂-Alkyl und insbesondere C₁- bis C₄-Alkylester), Aminomonomere und Nitrile, Vinyl- und Alkylvinylidene und Amide von ungesättigten Carbonsäuren genannt. Auch kommen ungesättigte Kohlenwasserstoff-Monomere umfassend Styrol-Verbindungen (bspw. Styrol, carboxyliertes Styrol und alpha-Methylstyrol), Ethylen, Propylen, Butylen und konjugierte Diene (Butadien, Isopren und Copolymere von Butadien und Isopren) in Frage. Bezüglich der Vinyl- und Halogenvinylidenmonomere seien Vinylchlorid, Vinylidenchlorid, Vinylfluorid und Vinylidenfluorid genannt. Beispiele für die Vinylester umfassen aliphatische Vinylester, wie etwa Vinylformat, Vinylacetat, Vinylpropionat, Vinylbutyrat, Vinylisobutyrat, Vinylvaleriat, Vinylcaproat und Allylester der gesättigten Monocarbonsäuren wie Allylacetat, Allylpropionat und Allyllactat. Hinsichtlich der Vinylether seien Methylvinylether, Ethylvinylether und N-Butylvinylether genannt. Typische Vinylketone umfassen Methylvinylketone, Ethylvinylketone und Isobutylvinylketone. Beispiele für die Dialkylester der monoethylenisch ungesättigten Dicarbonsäuren sind Dimethylmaleat, Diethylmaleat, Dibutylmaleat, Dioctylmaleat, Diisooctylmaleat, Dinonylmaleat, Diisodecylmaleat, Ditridecylmaleat, Dimethylfumarat, Diethylfumarat, Dipropylfumarat, Dibutylfumarat, Dioctylfumarat, Diisooctylfumarat, Didecylfumarat, Dimethylitaconat, Diethylitaconat, Dibutylitaconat und Dioctylitaconat. Insbesondere handelt es sich bei den monoethylenisch ungesättigten Monocarbonsäuren um Acrylsäure, Methacrylsäure, Ethacrylsäure und Crotonsäure. Bei den monoethylenisch ungesättigten Dicarbonsäuren seien Maleinsäure, Fumarsäure, Itaconsäure und Citronensäure genannt. Als monoethylenisch ungesättigte Tricarbonsäuren können im Hinblick auf die vorliegende Erfindung bspw. Aconitsäure und deren halogensubstituierte Derivate eingesetzt werden. Des Weiteren können die Anhydride und Ester der vorgenannten Säuren (bspw. Maleinsäureanhydrid und Citronensäureanhydrid) eingesetzt werden. Beispiele für Nitrile von ethylenisch ungesättigten Mono-, Di- und Tricarbonsäuren umfassen Acrylonitril, α-Chloracrylonitril und Methacrylonitril. Bei den Amiden der Carbonsäuren kann es sich um Acrylamide, Methacrylamide und andere α-substituierte Acrylamide und N-substituierte Amide bspw. N-Methylolacrylamid, N-Methylolmethylacrylamid, alkylierte N-Methylolacrylamide und N-Methylolmethacrylamide (bspw. N-Methoxymethylacrylamid und N-Methoxymethylmethacrylamid) handeln. Als Aminomonomere können substituierte und unsubstituierte Aminoalkylacrylate, Hydrochloridsalze der Aminomonomere und Methacrylate wie etwa β-Aminoethylacrylat, β-Aminoethylmethacrylat, Dimethylaminomethylacrylat, β-Methylaminoethylacrylat und Dimethylaminomethylmethacrylat eingesetzt werden. Im Rahmen der vorliegenden Erfindung seien hinsichtlich der kationischen Monomere α- und β-ethylenisch ungesättigte Verbindungen genannt, welche sich zur Polymerisation eignen und primäre, sekundäre oder tertiäre Aminogruppen enthalten, bspw. Dimethylaminoethylmethacrylat, Dimethylaminoneopentylacrylat, Dimethylaminopropylmethacrylat und tert.-Butylaminoethylmethacrylat oder organische und anorganische Salze dieser Verbindungen und/oder Alkylammonium-Verbindungen wie etwa Trimethylammoniumethylmethacrylatchlorid, Diallyldimethylammoniumchlorid, β-Acetamidodiethylaminoethylacrylatchlorid und Methaacrylamidopropyltrimethylammoniumchlorid. Diese kationischen Monomere können alleine oder in Kombination mit den vorgenannten weiteren Monomeren eingesetzt werden. Als Beispiele für hydroxyhaltige Monomere seien noch die β-Hydroxyethyl(meth)acrylate, β-Hydroxypropyl(meth)acrylate und γ-Hydroxypropyl(meth)acrylate genannt.

Als Diisocyanat sowie Alkohol oder Amin, mit einem fluorierten organischen Rest mit 1 bis 18 C-Atomen und 35 bis 85 Gew.-% Fluor können die bereits bei dem oben beschriebenen Verfahren verwendeten Verbindungen eingesetzt werden.

Handelt es sich bei der polymerisierbaren Gruppe in Formel (I) um Hydroxy- und/oder Aminogruppen, werden diese bevorzugt mit einer Isocyanat-Komponente zu Polymeren umgesetzt. Bei der Isocyanat-Komponente handelt es sich bevorzugt um eine aliphatische, cycloaliphatische, araliphatische und/oder aromatische Verbindung, bevorzugt um ein Diisocyanat oder Triisocyanat, wobei es sich auch um Mischungen dieser Verbindungen handeln kann. Hierbei ist es als bevorzugt anzusehen, dass es sich um Hexamethylendiisocyanat-1,6 (HDI), HDI Uretdion, HDI Isocyanurat, HDI Biuret, HDI Allophanat, 1-Isocyanato-3,3,5-trimethyl-5-isocyanatomethylcyclohexan (IPDI), 2,4- und/oder 2,6-Toluylendiisocyanat (TDI) und/oder 4,4'-, 2,4'- und/oder 2,2'-Diphenylmethandiisocyanat (MDI), Polymeres MDI, Carbodiimid-modifiziertes 4,4'-MDI, m-Xylendiisocyanat (MXDI), m- oder p-Tetramethylxylendiisocyanat (m-TMXDI, p-TMXDI), 4,4'-Dicyclohexylmethandiisocyanat (H12MDI), Naphthalin-1,5-Diisocyanat, Cyclohexan-1,4-diisocyanat, hydriertes Xylylen-diisocyanat (H6XDI), 1-Methyl-2,4-diisocyanato-cyclohexan, Tetramethoxybutan-1,4-diisocyanat, Butan-1,4-diisocyanat, 1,6-Diisocyanato-2,2,4-trimethylhexan, 1,6-Diisocyanato-2,4,4-trimethylhexan, 1-Isocyanato-1-methyl-4(3)-isocyanatomethylcyclohexan (IMCI) sowie 1,12-Dodecandiisocyanat (C12DI) handelt. Weiterhin kann es sich um 4-Dichlorophenyl diisocyanat, Dicyclohexylmethan-4,4'-diisocyanat, m-Phenylendiisocyanat, p-Phenylendiisocyanat, 4-Chloro-1,3-phenylendiisocyanat, 1,10-Decamethylenediisocyanat, Lysinalkylesterdiisocyanat, 3,3'-Dimethyl-4,4'-diphenylmethandiisocyanat, Xylylendiisocyanat, Tetramethylxylylendiisocyanat, 1,5-Tetrahydronaphthalendiisocyanat, Triisocyanatotoluol, Methylenbis(cyclohexyl)-2,4'-diisocyanat und 4-Methylcyclohexan-1,3-diisocyanat handeln. Weiterhin sind Polyisocyanate mit zwei oder drei Isocyanatgruppen pro Molekül geeignet. Es kann sich aber auch um Mischungen von Polyisocyanaten handeln, wobei die durchschnittliche NCO-Funktionalität der Isocyanatkomponente in der Mischung insbesondere bei 2,0 bis 2,3, 2,2 bis 2,4, 2,6 bis 2,8 oder 2,8 bis 3,0 liegen kann. Derivatisierte Polyisocyanate können ebenfalls verwendet werden, beispielsweise sulfonierte Isocyanate, blockierte Isocyanate, Isocyanurate und Biuret-Isocyanate.

Bevorzugt handelt es sich bei dem erfindungsgemäßen Polymer um ein Polyacrylat, Polymethacrylat, Polyurethan oder Polyharnstoff.

In einer besonders bevorzugten Ausführungsform wird das erfindungsgemäße Polymer zur Beschichtung einer Oberfläche verwendet, insbesondere bevorzugt zur permanenten wasser- und schmutzabweisenden Oberflächenbeschichtung.

Insgesamt wurde Überraschend gefunden, dass die polymerisierbaren Verbindungen der Formel (I), auch in geringen Einsatzmengen und bei einem geringeren Fluor-Gehalt, in den daraus hergestellten Beschichtungen zu hervorragenden wasser- und schmutzabweisenden Eigenschaften führen. Die aus der polymerisierbaren Verbindungen der Formel (I) hergestellten Produkte haben eine sehr gute mechanische und photochemischen Beständigkeit. Weiterhin weisen die hergestellten Beschichtungen einen guten optischen Gesamteindruck und eine hohe Härte sowie eine gute Chemikalienbeständigkeit auf. Insbesondere sind die erfindungsgemäßen fluorierten Monomere einfach und kostengünstig herstellbar.

Die nachfolgenden Beispiele verdeutlichen die Vorteile der vorliegenden Erfindung.

### Beispiele

### Beispiel 1

Herstellung des erfindungsgemäßen Fluormonomers 1 (F-Polycarbonat1000-Diol): 53,35 g Isophorondiisocyanat (IPDI) werden in einem 500 ml Dreihalskolben, ausgestattet mit Rührer, Rückflusskühler und Thermometer zusammen mit 0,04 g Dibutylzinn-dilaurat vorgelegt und auf 40°C temperiert. 120,00 g Polycarbonat (MW = 1000 g/mol) wird zugegeben, das Reaktionsgemisch wird auf 70°C erhitzt und für 1 Stunde bei 70°C gerührt. 2-(Perfluorohexyl)ethanol (DAIKIN A-1620) wird in zwei Portionen in einem Abstand von 20 Minuten (Gesamtmenge: 43,69 g) zugegeben. Nach jeder Zugabe des Fluoralkohols steigt die Temperatur des Reaktionsgemisches um ca. 10°C. Die Temperatur des Reaktionsgemisches wird auf 50°C eingestellt und nach einer Reaktionszeit von 30 Minuten wird der NCO-Gehalt überprüft. Ist der theoretische NCO-Gehalt erreicht, wird das Reaktionsgemisch langsam zu Diethanolamin (10,72 g) zugegeben. Der theoretische NCO-Gehalt nach der Isocyanat/OH-Reaktion beträgt 2,32 Gew.-% und der Festkörpergehalt 100 Gew.-%.

### Beispiel 2

Herstellung des erfindungsgemäßen Fluormonomers 2 (F-PolyTHF1000-Methacrylat): 85,45 g Isophorondiisocyanat (IPDI) werden in einem 500 ml Dreihalskolben, ausgestattet mit Rührer, Rückflusskühler und Thermometer zusammen mit 0,04 g Dibutylzinn-dilaurat vorgelegt und auf 40°C temperiert. 192,25 g PolyTHF (MW = 1000 g/mol) wird zugegeben, das Reaktionsgemisch wird auf 60°C erhitzt und für 1 Stunde bei 70°C gerührt. 2-(Perfluorohexyl)ethanol (DAIKIN A-1620) wird in drei Portionen in einem Abstand von 20 Minuten (Gesamtmenge: 70,00 g) zugegeben. Nach jeder Zugabe des Fluoralkohols steigt die Temperatur des Reaktionsgemisches um ca. 10°C. Die Temperatur des Reaktionsgemisches wird auf 50°C eingestellt und nach einer Reaktionszeit von 30 Minuten wird der NCO-Gehalt überprüft. Ist der theoretische Isocyanat-Wert erreicht, wird 26,24 g (2-Hydroxyethyl)-methacrylat (HEMA) zugegeben und die Reaktion bei 55°C bis zum Endpunkt (0% NCO) fortgesetzt (HEMA im Überschuss, 95% der OH-Gruppen werden umgesetzt). Der theoretische NCO-Gehalt nach der Isocyanat/OH-Reaktion beträgt 2,32 Gew.-%. Das Produkt wird noch mit Dipropylenglykol-Dimethylether (41,55 g) verdünnt, sodass der Festkörpergehalt 90 Gew.-% beträgt.

### Beispiel 3

Herstellung des erfindungsgemäßen Fluormonomers 3 (F-Polyglykol-Methacrylat): 42,74 g Isophorondiisocyanat (IPDI) werden in einem 500 ml Dreihalskolben, ausgestattet mit Rührer, Rückflusskühler und Thermometer zusammen mit 0,1 g Dibutylzinndilaurat vorgelegt und auf 40°C temperiert. 2-(Perfluorohexyl)ethanol (DAIKIN A-1620) wird in drei Portionen in einem Abstand von 20 Minuten (Gesamtmenge: 70,00 g) zugegeben. Nach jeder Zugabe des Fluoralkohols steigt die Temperatur des Reaktionsgemisches um ca. 10°C. Die Temperatur des Reaktionsgemisches wird auf 50°C eingestellt und nach einer Reaktionszeit von 30 Minuten wird der NCO-Gehalt überprüft. Ist der theoretische Isocyanat-Wert erreicht, wird 147,08 g MPEG MA 750 (Po-ly(ethylenglycol)methylethermethacrylat) zugegeben und die Reaktion bei 60°C bis zum Endpunkt (0% NCO) fortgesetzt (MPEG MA 750 im Überschuss, 98% der OH-Gruppen werden umgesetzt). Der theoretische NCO-Gehalt nach der Isocyanat/OH-Reaktion beträgt 7,16 Gew.-% und der Festkörpergehalt 100 Gew.-%.

### Beispiel 4

### Herstellung des Fluormonomers 4 (F-IPDI-HEMA) (Vergleichsbeispiel):

91,58 g Isophorondiisocyanat (IPDI) werden in einem 500 ml Dreihalskolben, ausgestattet mit Rührer, Rückflusskühler und Thermometer zusammen mit 0,04 g Dibutylzinn-dilaurat vorgelegt und auf 40°C temperiert. 2-(Perfluorohexyl)ethanol (DAIKIN A-1620) wird in drei Portionen in einem Abstand von 20 Minuten (Gesamtmenge: 150,0 g) zugegeben. Nach jeder Zugabe des Fluoralkohols steigt die Temperatur des Reaktionsgemisches um ca. 10°C. Die Temperatur des Reaktionsgemisches wird auf 45°C eingestellt und nach einer Reaktionszeit von 30 Minuten wird der NCO-Gehalt überprüft. Ist der theoretische Isocyanat-Wert erreicht, wird (2-Hydroxyethyl)-methacrylat (HEMA) zugegeben und die Reaktion bei 35°C bis zum Endpunkt (0% NCO) fortgesetzt (HEMA im Überschuss, nur 80-90% der OH-Gruppen werden umgesetzt). Der theoretische NCO-Gehalt nach der Isocyanat/OH-Reaktion beträgt 7,16 Gew.-% und der Festkörpergehalt 100 Gew.-%.

### Beispiel 5

### Allgemeine Herstellvorschrift für die Polyacrylate:

5,51 g Poly(ethyleneglycol)methylethermethacrylate (MPEG 475 MA), 38,20 g Dipropylenglykol-Dimethylether, 4,55 g Methacrysäure, 69,09 g Methylmethacrylat und 18,02 g Fluormonomer 2 werden in einem 250 ml Dreihalskolben, ausgestattet mit Rührer, Rückflusskühler, Thermometer unter Stickstoff vorgelegt und auf 75°C erhitzt. Die Reaktortemperatur wird auf 75°C eingestellt. Es werden 69,09 g 2-Mercaptoethanol zugegeben. 0,42 g Initiator (VAZO-67) wird in 16,61 g Toluol gelöst und die 3%ige Initiatorlösung wird linear über eine Zeit von 3h mit einer Perfusorpumpe mit einer Fließgeschwindigkeit von 5,44 ml/h zudosiert. Nach Dosierende wird 1 Stunde bei 70°C gerührt. Das Reaktionsgemisch wird abgekühlt und der pH-Wert mit NaOH (50%) auf 6,5-7 eingestellt. Die so erhaltene leicht trübe, weiße Lösung hat einen Feststoffgehalt von 64,30 Gew.-% und 2,6% Fluorgehalt im Feststoff.

### Kontaktwinkelmessung und Bestimmung der Oberflächenenergie:

Die Polyacrylate (Beispiele 6 und 7) wurden mit einem 250 µm Rakel auf eine Präsentationsfolie aufgerakelt. Die lösemittelhaltigen Filme wurden vor der Messung der Oberflächenenergie und des Vor- und Rückzugswinkels gegen Maschinenöl 3 Stunden unter Vakuum gelagert.

**Tabelle 1: Zusammensetzung der Perfluoralkylalkylgruppen-haltigen Polycarboxylatether der Beispiele 6 und 7 in Gew.-% und Kennzahlen**

| | Beispiel 6 (Fluormonomer 2) | Beispiel 7 (Vergleichsbeispiel; Fluormonomer 4) |
|---|---|---|
| MPEG-Methacrylat | 5,45 | 4,78 |
| Methylmethacrylat | 68,32 | 75,80 |
| Methacrylsäure | 4,50 | 4,99 |
| Fluormonomer | 17,82 | 6,86 |
| Fluorgehalt | 2,5 | 2,5 |
| FK theor | 71,66 | 69,02 |
| FK NP | 63,23 | 58,29 |
| Viskosität [mPas] | 45860 | 20450 |
| M_{w} | 3322 | 1969 |
| Mₙ | 1501 | 1259 |
| Aussehen | trüb | trüb |
| Kontaktwinkel H₂O [°] | 98,1 | 94,0 |
| Kontaktwinkel CH₂I₂ [°] | 32,8 | 89,2 |

Erläuterungen zu Tabelle 1:
M_{w} = gewichtsmittleres Molekulargewicht, gemessen mit Gelpermeationschromatographie (GPC) gegen einen PEG-Standard
Mₙ = zahlenmittleres Molekulargewicht, gemessen mit Gelpermeationschromatographie (GPC) gegen einen PEG-Standard
FK NP = (Feststoffgehalt nach Beendigung der Polymerisationsreaktion)
Die Viskosität wurde bei 21°C mit einem Rotationsviskosimeter der Firma HAAKE, Typ CAP 2000, Spindel 4, RPM 12 gemessen.
Kontaktwinkel H₂O: gemessener Kontaktwinkel gegenüber Wasser
Kontaktwinkel CH₂I₂: gemessener Kontaktwinkel gegenüber Diiodmethan Vergleichsbeispiel: Fluormonomer 4 auf Basis 2-(Perfluorohexylethanol)/IPDI/HEMA

### Vor- und Rückzugswinkel gegen Maschinenöl:

Für Oberflächen, die schmutzabweisend sind und Flüssigkeiten abperlen lassen, ist die Ausbildung eines großen Rückschreitewinkels wichtig. Dieser gibt an, wie leicht sich eine einmal benetzte Fläche wieder entnetzen lässt. Bei ideal chemisch homogenen, glatten Oberflächen würde man zwischen Fortschreite- und Rückschreitewinkel keine große Hysterese erwarten. Das Auftreten einer Differenz ist ein Anzeichen für Heterogenitäten in der Morphologie oder Oberflächenchemie.

**Tabelle 2: Ergebnis der Fortschreite- und Rückschreitewinkel mit Maschinenöl**

| Beschichtung | Fortschreitewinkel | Rückschreitewinkel | Hysterese |
|---|---|---|---|
| Beschichtung Beispiel 6 | 99,5° | 18,9° | 80,6 |
| Beschichtung Beispiel 7 (Vergleichsbeispiel) | 84,9° | 15,8° | 69,1 |

Beispiel 6 zeigt deutlich erhöhte Fort- bzw. Rückschreitewinkel im Vergleich zu Beispiel 7 bei demselben Fluoranteil, was auf eine verbesserte Schmutzabweisung schließen lässt.

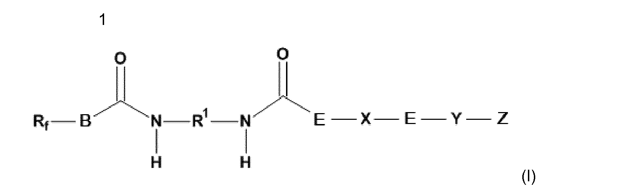

## Patentansprüche

1. Polymerisierbare Verbindung der Formel (I), 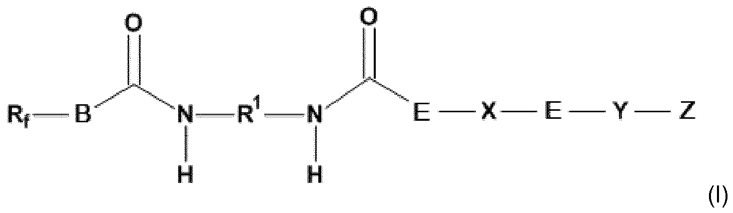 wobei
R_{f} für ein monovalentes fluoriertes organisches Radikal mit 1 bis 18 C-Atomen und 35 bis 85 Gew.-% Fluor,
R¹ für einen Rest aus der von acyclischem oder cyclischem Alkylen mit 5 bis 14 Kohlenstoffatomen, Arylen, Diarylenalkan und dialkylensubstituiertem Aryl gebildeten Gruppe
B für -O- oder -N(R²)-
R² für Wasserstoff, einen aliphatischen Kohlenwasserstoffrest mit 1 bis 12 C-Atomen oder ein monovalentes fluoriertes organisches Radikal mit 1 bis 18 C-Atomen und 35 bis 85 Gew.-% Fluor
X für ein polymeres Polyol mit mindestens zwei Wiederholungseinheiten und nach Entfernen von zwei OH-Gruppen
E für -O- oder -N(R³)-
R³ für einen Kohlenwasserstoffrest mit 1 bis 12 C-Atomen oder für ein Wasserstoffatom,
Y für eine chemische Bindung oder einen divalenten Rest mit 2 bis 20 C-Atomen und
Z für einen organischen Rest mit einer polymerisierbaren Baugruppe steht.

2. Polymerisierbare Verbindung nach Anspruch 1, **dadurch gekennzeichnet, dass** es sich bei X um eine Baugruppe mit der Formel (II) handelt, 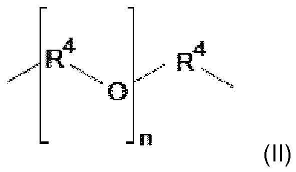 wobei R⁴ gleich oder verschieden sein kann und n eine ganze Zahl von 1 bis 70 repräsentiert.

3. Polymerisierbare Verbindung nach Anspruch 1, **dadurch gekennzeichnet, dass** es sich bei X um eine Baugruppe mit der Formel (IV) handelt, 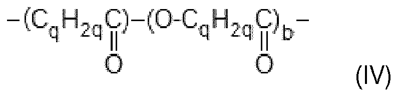 wobei q unabhängig voneinander für jede (C_{q}H_{2q}(C=O))-Einheit gleich oder verschieden für eine ganze Zahl von 2 bis 18 und b für eine ganze Zahl zwischen 1 und 30 steht.

4. Polymerisierbare Verbindung nach einem der Ansprüche 1 bis 3, **dadurch gekennzeichnet, dass** R¹ ausgewählt ist aus der Gruppe Methylen-, Ethylen-, Propylen-, Butylen-, Pentamethylen-, Hexamethylen-, Octamethylen-, 2-Ethylhexamethylen-, Phenylen-, Toluylen-, Methylenbisphenyl-, Propylenbisphenyl-, Cyclohexylen-, Methylen- oder Propylenbiscyclohexyl-Reste und einer Gruppe die sich durch Entfernen der Isocyanatgruppen aus 1-Isocyanato-3,3,5-trimethyl-5-isocyanatomethyl-cyclohexan ergibt.

5. Polymerisierbare Verbindung nach einem der Ansprüche 1 bis 4, **dadurch gekennzeichnet, dass** Y ausgewählt ist aus der Gruppe Methylen-, Ethylen-, Propylen-, Butylen-, Pentamethylen-, Hexamethylen-, Octamethylen-, 2-Ethylhexamethylen-, Phenylen-, Toluylen-, Methylenbisphenyl-, Propylenbisphenyl-, Cyclohexylen-, Methylen- oder Propylenbiscyclohexyl-Reste und einer Gruppe die sich durch Entfernen der Isocyanatgruppen aus 1-Isocyanato-3,3,5-trimethyl-5-isocyanatomethyl-cyclohexan ergibt.

6. Polymerisierbare Verbindung nach einem der Ansprüche 1 bis 5, **dadurch gekennzeichnet, dass** es sich bei R_{f} um mindestens einen Rest der allgemeinen Formel (V) handelt,
CF₃-(CF₂)_{z}-(CH₂)_{y}- (V)
wobei
z gleich oder verschieden für eine ganze Zahl von 0 bis 16 und
y gleich oder verschieden für eine ganze Zahl von 1 bis 6 steht.

7. Polymerisierbare Verbindung nach einem der Ansprüche 1 bis 6, **dadurch gekennzeichnet, dass** Z ein Rest aus der Reihe Acrylat, Methacrylat, Oxyalkylacrylat, Oxyalkylmethacrylat, Dialkanolaminyl, Oxyalkylcaprolactonacrylat, Oxyalkylcaprolactonmethacrylat, Oxycyloalkylmethacrylat, Oxycyloalkylacrylat und Oxyalkylmaleinsäureimid ist.

8. Polymerisierbare Verbindung nach einem der Ansprüche 1 bis 7, **dadurch gekennzeichnet, dass** es sich um eine Verbindung aus der Reihe (VI), (VII), (VIII) oder (IX) handelt, 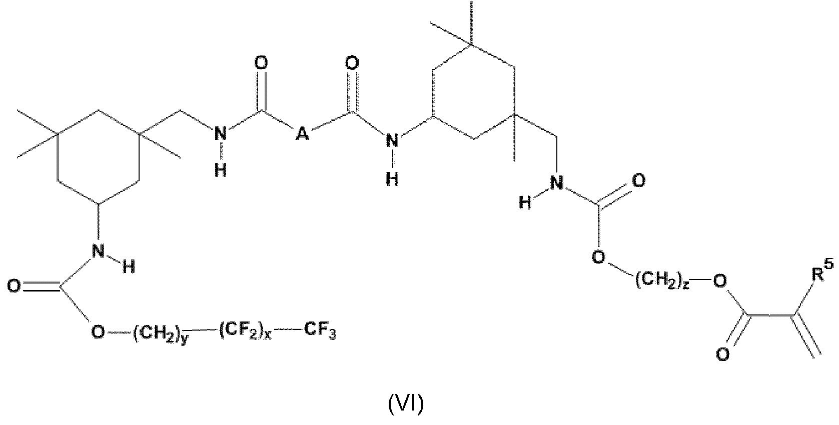 wobei
A für eine Baugruppe mit der Formel -O-(CₘH₂ₘO)ₐ-, mit m unabhängig voneinander für jede (CₘH₂ₘO)-Einheit gleich oder verschieden für eine ganze Zahl von 2 bis 4 und a für eine ganze Zahl von 2 bis 30
R⁵ für Wasserstoff oder Methyl
z für eine ganze Zahl von 1 bis 5
x für eine ganze Zahl von 0 bis 9
y für eine ganze Zahl von 2 bis 12
steht, 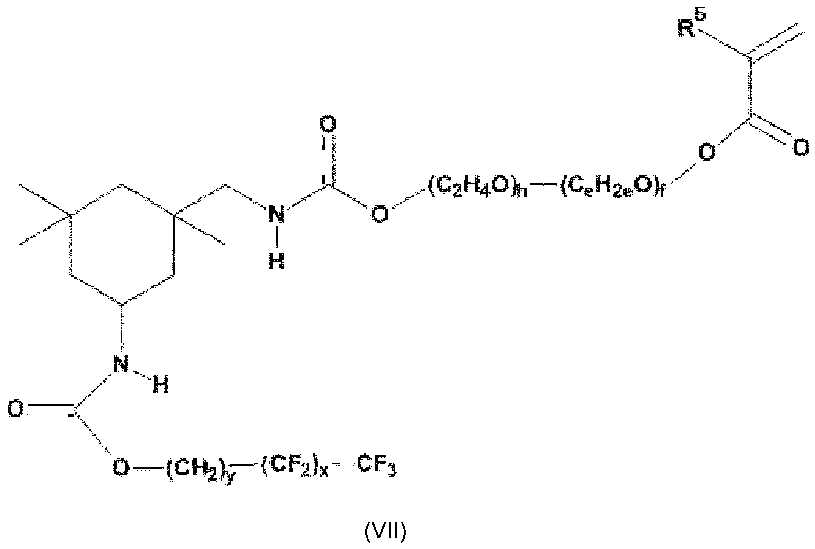 wobei
R⁵ für Wasserstoff oder Methyl
e unabhängig voneinander für jede (CₑH₂ₑO)-Einheit gleich oder verschieden für 2 oder 3
f für eine ganze Zahl von 0 bis 150
h für eine ganze Zahl von 0 bis 50, wobei die Summe aus f und h mindestens 3 ist
x für eine ganze Zahl von 0 bis 9
y für eine ganze Zahl von 2 bis 12
steht, 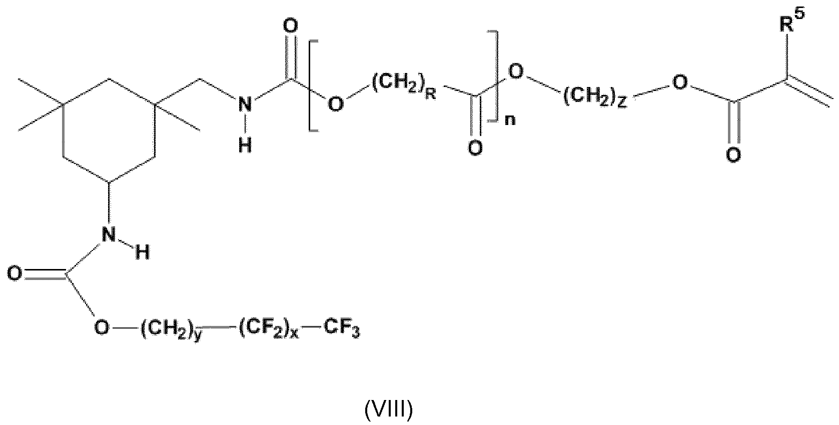 wobei
Z für eine ganze Zahl von 1 bis 50
R für eine ganze Zahl von 2 bis 18
n für eine Zahl von 1 bis 10
x für eine ganze Zahl von 0 bis 9
y für eine ganze Zahl von 2 bis 12
steht 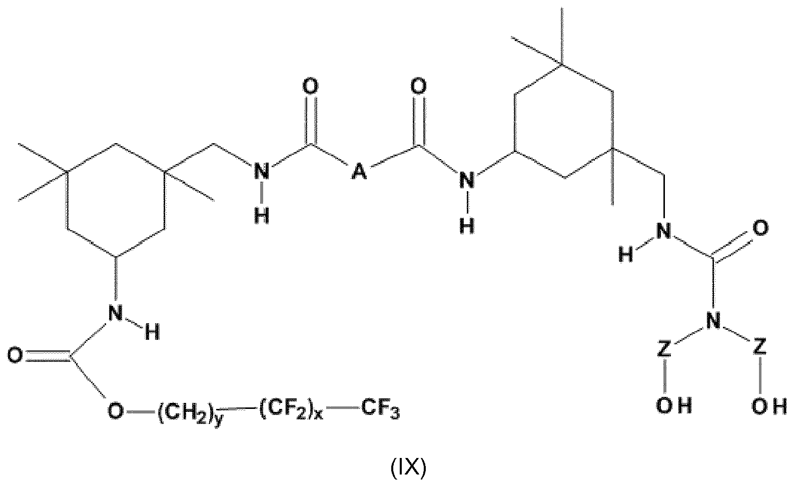 wobei
A für eine Baugruppe mit der Formel -O-(CₘH₂ₘO)ₐ -, mit m unabhängig voneinander für jede (CₘH₂ₘO)-Einheit gleich oder verschieden für eine ganze Zahl von 2 bis 4 und a für eine ganze Zahl von 2 bis 30
Z für eine (CₚH₂ₚ)-Einheit,
p für eine ganze Zahl von 2 bis 8
x für eine ganze Zahl von 0 bis 9
y für eine ganze Zahl von 2 bis 12
steht.

9. Verfahren zur Herstellung einer polymerisierbaren Verbindung nach einem der Ansprüche 1 bis 8, wobei
(a) mindestens ein polymeres Polyol mit mindestens zwei Wiederholungseinheiten und mindestens zwei Hydroxygruppen mit mindestens einem Diisocyanat
umgesetzt wird und das Produkt nachfolgend mit
(b) mindestens einem Alkohol oder Amin, mit einem fluorierten organischen Rest mit 1 bis 18 C-Atomen und 35 bis 85 Gew.-% Fluor,
umgesetzt wird und nachfolgend das Produkt mit
(c) einer organischen Verbindung mit einer polymerisierbaren Baugruppe umgesetzt wird.

10. Verfahren zur Herstellung einer polymerisierbaren Verbindung nach einem der Ansprüche 1 bis 8, wobei
(a) mindestens ein Alkohol oder Amin, mit einem fluorierten organischen Rest mit 1 bis 18 C-Atomen und 35 bis 85 Gew.-% Fluor, mit mindestens einem Diisocyanat umgesetzt wird und das Produkt nachfolgend mit
(b) mindestens einem polymeren Polyol, mit mindestens zwei Wiederholungseinheiten sowie mit mindestens einer Hydroxygruppe und einem polymersierbaren Rest
umgesetzt wird.

11. Polymer, umfassend ein Monomer nach einem der Ansprüche 1 bis 8.

12. Polymer nach Anspruch 11, **dadurch gekennzeichnet, dass** es sich um ein Polyacrylat, Polymethacrylat, Polyurethan oder Polyharnstoff handelt.

13. Verwendung eines Polymers nach einem der Ansprüche 11 oder 12 zur Beschichtung einer Oberfläche.

14. Verwendung eines Polymers nach Anspruch 13, zur permanenten wasser- und schmutzabweisenden Oberflächenbeschichtung.
